Europäisches Patentamt

European Patent Office

Office européen des brevets

(19)

(11) **EP 1 304 116 A1**

(12)

# EUROPEAN PATENT APPLICATION
published in accordance with Art. 158(3) EPC

(43) Date of publication:
23.04.2003 Bulletin 2003/17

(21) Application number: 00970347.1

(22) Date of filing: 13.07.2000

(51) Int Cl.[7]: **A61K 38/17**, A61K 35/12,
A61K 35/14, A61K 35/407

(86) International application number:
PCT/RU00/00295

(87) International publication number:
WO 02/004014 (17.01.2002 Gazette 2002/03)

(84) Designated Contracting States:
AT BE CH CY DE DK ES FI FR GB GR IE IT LI LU
MC NL PT SE

(71) Applicant: Endo-Farm-A, Zakrytoe Aktsionernoe
Obschestvo, Proizvodstvennoe Predpriyatie
Moskovskaya obl., 141100 (RU)

(72) Inventors:
• YAMSKOVA, Viktoria Petrovna
117463, Moscow (RU)

• YAMSKOV, Igor Alexandrovich
117463, Moscow (RU)
• RYKOV, Alexei Vasilievich
141100, Moskovskay obl. (RU)

(74) Representative: Lucking, David John et al
FORRESTER & BOEHMERT
Pettenkoferstrasse 20-22
80336 München (DE)

(54) **NEW CLASS OF BIOACTIVE GLYCOPROTEIN**

(57) The invention relates to a bioactive chemical composition, more specifically to proteins and can be used in medicine, veterinary and cell biology. The invented glycoproteins are extracted with the help of isoelectric focusing from intercellular space of tissues taken from different organs, blood serum and bile of the vertebrates (human beings and animals). Said glycoproteins have high biological activity in ultra low doses at concentration ranging from $10^{-12}$ to $10^{-29}$ mol/liter and lower.

**EP 1 304 116 A1**

**Description**

The invention relates to preparative and technological biochemistry and represents the obtaining of bioactive chemical composition. The invention can be used in cell biology, medicine and veterinary.

PREVIOUS LEVEL OF TECHNIQUE

Glycoproteins are conjugated proteins containing a protein part and a nonprotein component, organic or inorganic, which can be covalently, heteropolarly or coordinately connected to polypeptide chain and together with amino acids is present in hydrolysate. The prosthetic part of glycoproteins can be represented by neutral saccharides (galactose, mannose, and fucose) or by amino saccharides (N-acetylglucosamine, N-acetylgalactosamine or acidic derivatives of monosaccharides) (H.-D. Jacubke, X. Ashkite "Amino acids, peptides, proteins" Moscow, Mir 1985 p. 345).

Glycoproteins are wide spread in nature. Major components of blood serum (immunoglobulins, transferrins, etc.), group substances of blood, antigens of different viruses (influenza, measles, etc.), some hormones, lectins, enzymes, etc. belong to glycoproteins.

Lectins represent a large group of glycoproteins. Protein part of their molecule is characterized by the absence of serine and threonine remains, which carry out the linkage of carbohydrate component of lectin's molecule with a polypeptide chain. The average content of carbohydrates in lectins makes about 5 %. The composition of carbohydrates is basically limited to the remains of galactose, mannose, fucose and N-acetylglucosamine H.-D. Jacubke, X. Ashkite "Amino acids, peptides, proteins" Moscow, Mir 1985 p. 428-429).

DESCRIPTION OF EXISTING METHODS

Glycoprotein with immunosuppressive ability is known from GB 2078229, 1982. Glycoprotein is obtained with the help of isoelectric focusing of blood serum or ascitic fluid taken from a human being or warm-blooded animal and subsequent fraction selection with isoelectric point (đI) in đI interval 2.6-3.6. This glycoprotein is used in patients that undergone transplantation to suppress the foreign body reaction.

According to the patent GB 2095260, 1982 a bioactive substance - glycoprotein with molecular weight 3000 - 5000 - is received. It has the ability to inhibit the reproduction of toxoplasma in homologous and heterologous cells.

Glycoprotein with anticancer activity is known from the patent GB 2117385, 1982. Molecular weight of this glycoprotein is 7000 - 90000; saccharides content is 8 - 45 %, including 6 - 28 % of hexose, 1 - 11 % of hexosamine and 1-6 % of sialic acids. It is stable in water solution with đI =2.0, 7.0 or 11.0 at 4°Ñ for 24 hours or more, and in water solution with đI =7.0 at 60°Ñ - for 3 hours or more. This glycoprotein selectively affects cancer cells, without affecting normal cells.

Immunological glycoprotein is known from the patent ÑÍ 634334, 1983: It contains protein part 89±4%, carbohydrate part 11.1±2.2%, hexose 5.3±1.1%, N-acetyl residue of hexosamine 2.8±0.5%, and N-acetyl residue of neuraminic acid 2,9±0,5%. Molecular weight of this glycoprotein is 32000±6000; đI=4.3±0.3; sedimentation factor is S20n=3.2±0.3.

Serum glycoprotein with molecular weight 12.5 kDa is known from the patent RU 2136695, 1999. It is biologically active in low doses and contains about 50 % of carbohydrate remains. Isoelectric point lies in đI interval from 4.6 to 4.7.

New glycoprotein is known from the patent ÅĐ 000134,1978. It contains $\alpha$-amino acid 75±6 %, carbohydrate part 24.6±5.2 %, hexosan 2.9±2 %, N-acetyl residue of hexosamine 7.1±1.5 %, fucose 0.2±0.2 %, and N-acetyl residue of neuraminic acid 8.4±1.5 %. Molecular weight of this glycoprotein is 65000±10000, đI=3.4±0.4.

Homogeneous glycoprotein with molecular weight 56 kDa was extracted and studied from FVO and Ceneva P. falciparum. Its isoelectric point lies in đI 5.5. This glycoprotein contains N-acetylgalactosamine and mannose in carbohydrate part (patent US 4835259, cl. Ñ07Ê15/15, 1989).

Closest to this given invention is glycoprotein with molecular weight 5 - 300 kDa, isoelectric point lying in đI interval from 2.5 to 5.0, weight ratio of protein and carbohydrate parts 50:50 - 80:20, containing in carbohydrate part the remains of fucose, ribose, arabinose, xylose, mannose, galactose, glucose and glucosamine, and in protein part the remains of aspartic and glutamic acids, threonine, serine, proline, glycine, alanine, cysteine, valine, methionine, cystathionine, isoleucine, leucine, tyrosine, phenyl alanine, tryptophan, ornithine, lysine, histidine, arginine (US 4683438, cl. Ñ07Ê15/14, 1987).

BRIEF DESCRIPTION OF INVENTION

Thus, this invention relates to glycoproteins having biological activity in ultra low doses, which can be used in medicine and pharmaceutical industry.

On the other hand, this invention relates to the obtaining of glycoprotein with biological activity in ultra low doses and its application as medicinal preparation.

Thus, technical task of the invention is the obtaining of glycoprotein with biological activity in ultra low doses ($10^{-12}$ to $10^{-29}$ mol/liter and lower).

This specified technical result is achieved by new glycoproteins, which are extracted with the help of isoelectric focusing from intercellular space, blood serum, bile and tissues taken from different organs of the vertebrates (human beings and animals).

They are soluble in saturated (100%) solution of ammonium sulphate; their apparent molecular weight is 10-45 kDa and they have biological activity in ultra low doses. Solutions of glycoproteins in concentration of $10^{-12}$ - $10^{-18}$ mol/liter completely preserve biological activity at multiple freezing-and-unfreezing, and also after heating at 100°Ñ during 10 minutes.

Molecular weight of glycoproteins has been estimated with the help of electrophoresis method in polyacrylamide gel (PAAG) with sodium dodecylsulphate according to Lemly's method (see the technique applied). As molecular weight markers, a set made by LKB Company was utilized. It included 6 proteins with molecular weight from 94 kDa to 14.4 kDa. Apparent molecular weight of the glycoprotein taken from blood serum with đÍ lying in đÍ interval 4.65-5.1 (SG) was 35-37 kDa according to PAAG- phoresis and 25-27 kDa according to the data of gel - chromatography. Apparent molecular weight of the neutral glycoprotein taken from liver (đÍ in đÍ interval 6.8-7.2) (NGL) was 15 kDa and 22 kDa correspondingly. As far as acidic glycoprotein taken from liver (AGL) is concerned, its apparent molecular weight was determined only by gel - filtration method and was equal 17 kDa. (It was not possible to define molecular weight by PAAG-electrophoresis method because the acidic glycoprotein fraction could not be painted.)

For electrophoresis in polyacrylamide gel, a device for vertical electrophoresis ABGE-1 (Himifil, USSR) was used. Denaturing conditions were applied with detergent sodium dodecyl_sulfate (SDS) addition, according to Lemly's method. Thickness of gel was 0.75 mm, size was 115Õ115 mm, and maximal quantity of strips was 13. Separating gel (12.5 %) was prepared from the following components: distilled water (6.7 ml), 1.51 Tris-HCl with đÍ 8.8 (5.0 ml), 10 % solution of SDS (0.2 ml), 30 % solution of Acrylamide/N'N'-bis-methylene-acrylamide (8.0 ml), TEMED (10 µl), 10 % solution of ammonium persulphate (100 µl).

Concentrating gel (4 %) was prepared from the following components: distilled water (6.1 ml), 0.5Ì Tris-HCl with đÍ 6.8 (2.5 ml), 10 % solution of SDS (0.1 ml), 30 % solution of Acrylamide/N'N'-bis-methylene-acrylamide (1.3 ml), TEMED (10 µl), 10 % solution of ammonium persulphate (50 µl).

The electrode buffer: Tris-glycine with đÍ 8.3 and 0.1 % SDS. Frozen-dried sample was dissolved (about 0.1 mg/ml) in the following solution: distilled water (4.0 ml), 0.5 M Tris-HCl with đÍ 6.8 (1.0 ml), glicerol (0.8 ml), 10 % solution of SDS (1.6 ml), 2- mercaptoethanol (0.4 ml), 0.05 % solution of bromphenol dark blue (0.2 ml). Then, before applying on gel, samples were incubated for 3 - 5 minutes at 100°C. 10-15 µl of specimen were applied on one strip. Electrophoresis was carried out at constant voltage of 200 V. When concentrating gel was passed, voltage of 100 V was applied.

As molecular weight markers, a set made by LKB Company was utilized. It included phosphorylase b (94 kDa), bovine serum albumin (67 kDa), chicken egg albumin (45 kDa), carbonic anhydrase (30 kDa), soybean inhibitor of trypsin (20 kDa), and chicken lysozyme (14.4 kDa).

Painting of gels was carried out with the help of colloidal silver or by Kumassy stain G-250. For painting by colloidal silver gels were previously washed out in distilled water, then in water solution with 5 % of ethanol and 5 % of acetic acid within not less than three hours. Then, gel was quickly (for 5 minutes) washed out in distilled water and put in 10 % solution of glutaric aldehyde. Unreacted glutaric aldehyde was rinsed out during three-five washings (each washing 30 minutes long). Afterwards, gel was incubated in dithiotreitol solution (5 mg/l) during 30 minutes and then thoroughly rinsed with water. The washed gel was placed in $AgNO_3$ solution (0.1 g/l) for 30 minutes, then again thoroughly rinsed with water and after that placed in a developer before well-defined strips occurred. The developer was 37% solution of formalin in 3% aqueous $Na_2SO_3$ (50-100 µl of formalin/100 ml of solution). For interruption of development 3-5 ml of citric acid was added.

When painting with Kumassy stain G-250 was held, gel was fixed with the following mixture: trichloroacetic acid/methanol/water (10:40:50). Painting was carried out with 0.04 % solution of Kumassy stain G-250 in 3.5% perchloric acid.

Usually, blue protein strips appear in 5 minutes. To reduce background stain, gel can be placed in solution of acetic acid /methanol/water (10:40:50) for 1-3 hours.

Molecular weight of glycoproteins with the help of gel - penetrating HPLC method was determined on a column TSK G3000SW (300õ7.5mm). The elution was carried out by 100 mM $NaH_2PO_4$ at đÍ =7. For calibration the following witness proteins were used: ovotransferrin (78 kDa), bovine serum albumin (67 kDa), chicken egg albumin (45 kDa), carbonic anhydrase (30 kDa), soybean inhibitor of trypsin (20 kDa), myoglobin (17.2 kDa), and chicken lysozyme (14.3 kDa). These preparation were made by Serva Company (Germany) and Sigma Company (USA).

Also, under this invention, amino acidic composition and carbohydrate composition of glycoproteins and the presence of glycosylation were determined.

DEFINITION OF AMINO ACIDIC COMPOSITION

Definition of amino acidic composition was carried out on the amino acidic analyzer Hitachi 835 in A. N. Beloserskiy Institute of Physical-Chemical Biology. Separation was held on a chromatographic column with sulfo-polystyrene cations of 2613 mark. Detection was spectrophotometric in ninhydrin derivatives (wave length - 570 and 440 nm). Before the analysis, hydrolysis of proteins was conducted with the mixture of 12 n. Í Ñl/concentrated trifluoroacetic acid (2:1), with addition of 0.005 % mercaptoethanol during 1 hour at 155°Ñ.

DEFINITION OF CARBOHYDRATE COMPOSITION

Definition was carried out on carbohydrate analyzer Biotronic LC 2000 (Germany) on a column with sorbent Durrum DAX 8-11 (USA) at 70°C in 0.4 M borate buffer with đÍ 8.0. The size of a column was 3.7Õ75 mm. Detection was conducted at 570 nm with solution of 2, 2 '- bicinchonine of copper. Before definition, hydrolysis of 1 ml of tested substance (0.1 mg/ml) was carried out in 10 ml of 2 M trifluoroacetic acid for 3 hours at 100°Ñ.

DEFINITION OF GLYCOSYLATION PRESENCE

To define the presence of glycosylation, 5 µl of 0.1 M acetate buffer with đÍ 4.6 was added to 50 µl of protein solution in water (0.1 mg/ml), then the solution of sodium periodate in the same buffer (5-10 µl) was added so, that the concentration of periodate in the reaction mixture was 1-2 mM. After that the solution was left for 30 minutes at room temperature. Unreacted periodate was neutralized by 0.5Ì solution of sodium thiosulfate in water before the coloring disappeared. Then the solution of dinitrophenylhydrazine (DNPH) in dimethylsulfoxide (DMSO) was added up to concentration of DNPH in the reaction mixture became 2.5-5 mM. After that the solution was left for 30 minutes at room temperature.

Further, the resulted Shiff's bases were reduced with the help of the solution of sodium borane in DMSO (0.1 mg/ml).

The analysis of products was carried out by the method of gel-penetrating chromatography on the column TSK 2000 PW (5.6Õ300 mm) with the help of high pressure fluid chromatograph. Eluent was 50ì Ì acetated buffer with đÍ 4.5; rate was 0.5 ml/min; ultraviolet detection was at wave length of 365 nm. The following characteristics were estimated: retention time of DNPH, of intact glycoprotein (on absorption at 280 nm) and of modified DNPH glycoprotein (in case of the presence of absorption peak at 365 nm with retention time, approximately equal to retention time of the unmodified glycoprotein).

As modeling glycoproteins, the following were utilized: chicken egg albumin, ovomucoid, S - carboxy methylated mucin, rhodopsin-binding glycoprotein from egg yolk.

DEFINITION OF GLYCOPROTEIN GLYCOSYLATION UNDER THIS INVENTION

After tests on modeling glycoproteins with known structure, this method was applied to the extracted proteins. The results testify that all these substances are glycoproteins. The peak of studied glycoprotein is seen on the chromatogram, and the peak of unreacted DNPH is also seen. Time of modified glycoprotein outcome slightly differs from native, but that could have been expected.

For example, for a purified specimen of SG the carbohydrate composition analysis shown, that this given protein is strongly glycosylated. Its weight contains 40-55% of carbohydrates. From carbohydrates N-acetylglucosamine and mannose were detected in the ratio of 2:5. Knowledge of biological paths of glycoprotein synthesis allows assuming that, SG mainly contains N-bound oligosaccharide chains rich in mannose.

Other aspect of this invention is application of the described glycoproteins in preparation of various pharmaceutical compositions (drops, ointments, medical creams, gels etc.).

Pharmaceutical composition under this invention contains glycoprotein in effective amount, having biological activity in ultra low doses and being one of the aspects of the [given] invention, and pharmaceutically acceptable carrier (Examples 15-17).

Pharmaceutically acceptable carrier can be an organic carrier, polymeric (carbohydrates, cellulose), and inorganic carrier. Selection of the carrier is determined, first of all, by method of pharmaceutical composition application (prescription).

Example 15 describes pharmaceutical composition, which is bioregulator of reparative processes in epithelial and connecting tissues.

Glycoproteins under this given invention were extracted from the following tissues: liver, lung, thymus gland, spleen, heart, kidneys, pancreas, lactiferous and thyroid glands, intestines, testicles, ovaries, brain, marrow, eye tissues, and also from blood serum and bile.

The extraction process of the specified glycoproteins under this invention includes the following stages:

a) Obtaining the extracts from tissues of various human and animal organs;
b) Salting proteins out of the tissue extract;
c) The extraction itself (separation and purification) of glycoproteins by method of isoelectric focusing;
d) Collecting fractions with glycoprotein identification, defining their biological activity and biological effect.

Glycoproteins that we identified can be divided conditionally into three groups:

Acidic proteins migrating to the anode;
Proteins with đÍ lying in đÍ interval from 4.6 to 8.5 (subacid, neutral and subbasic);
Basic proteins migrating to the cathode.

Method of isoelectric focusing is one of the traditional methods of biopolymer extraction and purification (separation). Glycoproteins under this present invention belong to biopolymers. This method is used in physical and analytical chemistry.

It is based on the following: external electrical field creates a stable đÍ gradient, and value of đÍ grows from the anode to the cathode. In such system every protein moves in this or that direction according to its positive or negative charge until it reaches that area where đÍ value coincides with this protein's isoelectric point (đI). In this area the protein terminates its further moving under electrical field, since its charge becomes 0.

The applied electrical field, which is supporting a stable gradient of đÍ, prevents the zone from diffusive washing. To create a stable gradient đÍ, special substances are used named ampholincs.

Isoelectric focusing was carried out in saccharose gradient, using the column LKB-440 (LKB, Sweden) and ampholincs (Serva, Sweden) with đÍ range 3.5- 10.0, at 4°Ñ during 100 hours and at voltage of 500 - 1500 V. Supernatant in the form of solution was entered in heavy gradient solution in amount not more than 100 mg of general protein. Detection of fractions was carried out by spectrophotometry at 280 nm. Dialysis of fractions after isoelectric focusing was conducted in bags made from cellulose film against distilled water. The following fractions were collected:

1. Acidic glycoproteins migrating to the positively charged electrode;
2. Glycoproteins with đI lying in đÍ interval from 4.6 to 8.5 (subacid, neutral and subbasic);
3. Basic glycoproteins migrating to the negatively charged electrode.

Glycoproteins of three specified groups were extracted from tissues of the following organs: liver, lung, thymus gland, spleen, heart, kidneys, pancreas, lactiferous and thyroid glands, intestines, testicles, ovaries, brain, marrow, lens, cornea, eye pigmented epithelium, retina, and also from blood serum and bile.

All identified glycoproteins displayed biological activity in ultra low doses at concentration of $10^{-12}$ to $10^{-29}$ mol/liter and lower.

Biological effect ($E_a$) was defined for extracted glycoproteins under this given invention. It was calculated, for example, by the following formula:

$$E_a = 200\% - N_t/N_c \times 100\%$$

Where $E_a$ is biological effect, caused by glycoprotein,
$N_t$ and $N_c$ is the amount of cell nuclei, released from 1 mg of tissue in the test (tissue culture with glycoprotein) and in the control (tissue culture without glycoprotein) correspondingly.

Calculation of cell nuclei was carried out in defined volume of Goryaev's chamber. Statistical data processing was conducted by method of variational statistics with the usage of Student's criterion. Procedure of tissue culture preparation consists of the following. After the decapitation of an animal (mice-hybrids ÑÂÀ/Ñ57ÂI, males, 16-18 g), liver was placed in 199 medium at room temperature. Fragments of tissue with the size of 1-1.5 $mm^2$ were cut out from central part of large liver lobe, but the edges of large liver lobe and the area with large blood vessels was not taken.

The cultures were placed in seated in penicillin vials, 5 tissue cultures in each vial, with nutrient medium of the following composition: 1 ml of 199 medium + 0.2 ml of cattle serum or horse serum + 0.1 ml of studied glycoprotein solution in certain concentration. In control vials 0.1 ml of Ringer's solution was added instead of glycoprotein solution. All vials were incubated at 37°C within 2 hours.

To estimate the parameter describing viscoelastic properties of hepatocyte membrane, each tissue fragment was dried with filter paper, then placed in special glass homogenizer with a backlash of 50 microns, after that 0.1 ml of 0.1 % solution of trypan dark blue, prepared on Ringer's solution, was added, and finally, this solution was dispersed, rotating a pestle 25-30 times. Then the amount of single cell nuclei, resulted in dispersion, was calculated. Biological effect of glycoprotein was calculated by the above-mentioned formula.

Glycoproteins of three specified groups that influenced viscoelastic properties of hepatocyte membrane were extracted from tissues of the following organs: liver, lung, thymus gland, spleen, heart, kidneys, pancreas, lactiferous and thyroid glands, intestines, testicles, ovaries, brain, marrow, lens, cornea, eye pigmented epithelium, retina, and also from blood serum and bile.

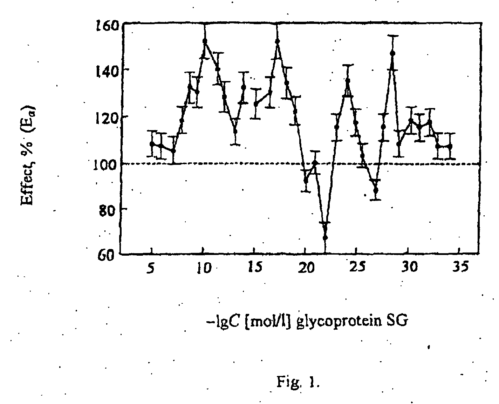

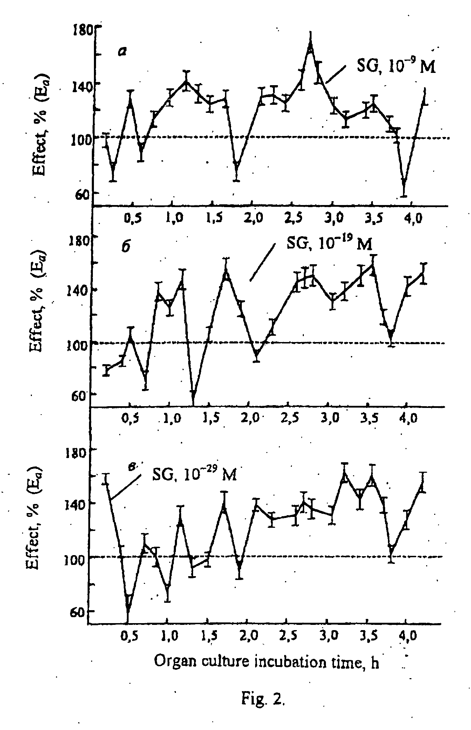

For identified glycoproteins value of $E_a$ was from 125 up to 150 % for concentration of $10^{-12}$ to $10^{-29}$ mol/liter and lower (fig. 1, 2).

DETAILED DESCRIPTION OF INVENTION EMBODIMENT

Glycoproteins under this present invention that display biological activity in ultra low doses, are extracted from intercellular space, blood serum, bile and tissues taken from different organs: liver, lung, thymus gland, spleen, heart, kidneys, pancreas, lactiferous and thyroid glands, intestines, testicles, ovaries, brain, marrow, lens, cornea, eye pigmented epithelium and retina using the above-mentioned stages.

The following examples illustrate, but by no means limit this given invention.

Example 1

Glycoproteins from mammalian liver

1.1. Obtaining a liver extract

The experiment was carried out on rats of Wistar line, both male and female; with weight of 150-180 g. Animals were decapitated. Within 30-40 seconds liver of every animal was washed from blood through Portal vein.

It was done with perfusion solution flow (0.15 mol/liter of NaCl; 0.04 mol/liter of ÊÑI; 0,001 mol/liter of $CaCl_2$) with rate 5-7 ml/ min. The liver was cut on the fragments with weight of 1.5-2.0g and placed in the solution with above-mentioned composition at 4° C for 2 hours (15-20 ml of the solution for 1 liver).

The obtained extract was collected. The tissue was flooded with fresh portion of physiological solution and extracted for 1 more hour. The obtained extracts were combined together. For elimination of blood cells and damaged liver cells, tissue extract was centrifuged at 5000g within 20 minutes, and then it was decanted and used for further purification.

1.2. Salting proteins out from the tissue extract

Dry ammonium sulphate was gradually added to the tissue extract with intensive stirring before obtaining the saturated salt solution (4°C, đÍ 8.0-8.5). The formed precipitate of admixture proteins was precipitated by centrifuging at 35000g during 30 minutes. Supematant was collected and dialyzed for a long period of time against distilled water, using a cellulose film of domestic production (GOST 7730-89). During dialysis distilled water was repeatedly replaced by fresh water. After complete elimination of ammonium ions, supematant was concentrated up to volume of 100ml, with the help of lyophilic desiccation method, and the procedure of salting-out was repeated under the same conditions. Similarly obtained the second supernatant was also dialyzed against distilled water before complete elimination of ammonium ions, and then was separated by isoelectric focusing method.

Isoelectric focusing of tissue extract supernatant was carried out according to the technique described above.

Example 2

Glycoproteins from mammalian thymus gland

The experiment was carried out on rats of Wistar line, both male and female; with weight of 150-180 g. Animals were decapitated. Thymus glands were cut out, thoroughly rinsed in physiological solution (0.15 mol/liter of NaCl; 0.04 mol/liter of ÊÑi; 0,001 mol/liter of $CaCl_2$) and extracted in the solution with above-mentioned composition at 4°C for 2 hours (3-4 ml of the extracting solution for 1 thymus gland). The obtained extract was collected. Thymus glands were flooded with fresh portion of physiological solution and extracted for 1 more hour. The obtained extracts were combined together. For elimination of blood cells and damaged thymus cells, tissue extract was centrifuged at 5000g within 20 minutes, and then it was decanted and used for further purification. Salting-out and đÍ -isoelectric focusing of tissue extract were carried out according to the technique described above. After đÍ -isoelectric focusing, the same way as separating liver extract, three protein fractions were collected: acidic, with đI in đÍ interval 4.6-8.5 and basic glycoproteins.

All identified thymus gland glycoproteins displayed biological activity in ultra low doses at concentration of $10^{-12}$ to $10^{-29}$ mol/liter and lower.

Example 3

Glycoproteins from mammalian eye tissues

From fresh bull eyes (30 pieces) the following tissues were extracted: lens, cornea, pigmented epithelium and retina. The tissues were thoroughly rinsed in physiological solution (0.15 mol/liter of NaCl; 0.04 mol/liter of ÊÑI; 0,001 mol/liter of $CaCl_2$), cut in large pieces and extracted separately in the solution with above-mentioned composition at +4°C for 2 hours. The obtained extracts were centrifuged at 3000g within 15 minutes, and then they were decanted and used for further purification. Salting-out and đÍ - isoelectric focusing of tissue extract were carried out according to the technique described above. After đÍ -isoelectric focusing, three protein fractions for each tissue were collected: acidic, with đI in đÍ interval 4.6-8.5 and basic glycoproteins of lens, comea, pigmented epithelium and retina.

All identified glycoproteins from eye tissues displayed biological activity in ultra low doses at concentration of $10^{-12}$ to $10^{-29}$ mol/liter and lower.

Example 4

Extraction of glycoprotein with đI in đÍ interval 4.65-5.1 from cattle blood serum

Dry ammonium sulphate was gradually added to 5 l of cattle blood serum before obtaining the saturated salt solution (4°C, đÍ 8.0-8.5). The formed precipitate was deleted by filtering and the filtrate was centrifuged at 10000g during 15 minutes. Then protein sediment was removed, supernatant was collected and dialyzed for a long period of time against distilled water before complete elimination of ammonium ions. Then it was lyophilized and dissolved again in distilled water up to volume of 0.5 l. To obtained solution dry ammonium sulphate was added with stirring before saturation. This mixture was left for 4 days at 4°Ñ, and then it was centrifuged at 10000g during 15 minutes. Supernatant was dialyzed against distilled water before complete elimination of ammonium ions. Then it was lyophilized and dissolved again in 50 ml of distilled water. Further separation was carried out by đÍ -isoelectric focusing method according to the technique described above.

After đÍ -isoelectric focusing the fraction in đÍ interval 4.65-5.1 was collected, dialyzed against distilled water and lyophilized. 0.55 mg was received. The obtained specimen was finely divided powder of homogeneous glycoprotein, slightly painted in cream color. Glycoprotein contains 40-55% of carbohydrates. The molar ratio of D-mannose to N-acetyl-D-glucosamine makes from 5:2 to 3:2, biological activity (influence on viscoelastic properties of hepatocyte membrane in vitro) at glycoprotein concentration of $10^{-12}$ to $10^{-29}$ mol/liter and lower makes not less than 125 % in relation to the control.

Similarly glycoprotein is extracted from rat blood serum, dog blood, horse blood and human blood. All glycoproteins extracted from blood serum have isoelectric point in đÍ interval 4.65-5.1, apparent molecular weight of 35-37 kDa according to SDS-electrophoresis in polyacrylamide gel and 25-27 kDa according to gel-chromatography. They have biological activity in ultra low doses at concentration from $10^{-12}$ to $10^{-29}$ mol/liter and lower.

COMMERCIAL APPLICATION

The following examples (5-17), drawings (1-29) and tables (1-8) illustrate biological activity and biological effect of glycoproteins discussed in this given invention.

Example 5

Influence of glycoproteins on viscoelastic properties of hepatocyte membrane in vitro

The research was carried out with the help of multiple organ cultivation of liver tissue fragments with weight of 1 - 1.5 mg, in nutrient medium, where certain volume of the studied glycoprotein solution was added. For each glycoprotein an interval of concentration was defined, in which its biological activity is displayed. For this purpose a series of consecutive dilution of initial specimen (concentration of 0.1 mg of protein/ml) in 10, 100, 1000 etc. times to $10^{-15}$ mg of protein/ml was conducted. In control vials the same volume of Ringer's solution was added instead of studied specimen. All explantats studied in one experiment were received from one animal's liver. In each separate experiment, not less than 5 fragments of tissue were taken in consideration for each experimental point (appropriate concentration of glycoprotein solution). Not less than 3-4 experiments were carried out to define biological activity of each glycoprotein.

Biological effect was calculated by the above-mentioned formula, and is illustrated in fig. 1-2.

### Example 6.

Influence of thymus gland basic glycoprotein on delayed-type hypersensitivity reaction

Basic thymus glycoprotein (BTG) was administered to four groups of 5 mice with C57BL/6 spf status, with weight 20g. Intraperitoneal introduction was made three times in doses of $2 \times 10^{-6}$, $2 \times 10^{-12}$, $2 \times 10^{-16}$ and $2 \times 10^{-18}$ g for one animal within three days, from the moment of intraperitoneal immunization of mice with sheep erythrocytes in dosage of $2 \times 10^8$ for one animal. In 5 days after immunization, $1 \times 10^8$ of sheep erythrocytes were administered to the recipients in 50 μl subcutaneously in back leg's foot. And in one day more, the intensity of edema was defined in comparison with counter-lateral extremity by Kitamura method, and number of spleen antibody-forming cells was defined by Jeme method. The results are shown in table 1.

Table 1

| Influence of thymus gland basic glycoprotein on antibody-forming and delayed-type hypersensitivity reaction in mice of C57BL/6 line | | |
|---|---|---|
| Test groups | Delayed-type hypersensitivity reaction (%) | Antibody-forming cells to spleen |
| The Control | $25,2 \pm 1,61$ | $5900 \pm 1500$ |
| BTG, $2 \times 10^{-6}$g | $25,22 \pm 2,5$ | $3350 \pm 1600$ |
| BTG, $2 \times 10^{-12}$g | $32.88 \pm 1,31$** | $7600 \pm 1500$ |
| BTG, $2 \times 10^{-16}$g | $29,5 \pm 2,59$ | $7360 \pm 440$ |
| BTG, $2 \times 10^{-18}$g | $30,1 \pm 0,8$* | $4450 \pm 900$ |

The results represent arithmetic average ± average mistake.
** - Reliable distinctions from the Control, Đ < 0.001
*- Đ <0.05

It is well seen from the table, that the specimen doesn't exert a great influence on antibody-forming cells formation in spleen. At the same time, the specimen introduction in dose of $2 \times 10^{-12}$ g for one animal results in highly reliable intensifying of delayed-type hypersensitivity reaction.

Thus, we show the ability of BTG specimen in dose of $2 \times 10^{-12}$ g for one animal to stimulate delayed-type hypersensitivity reaction, caused by the activity of Ò-lymphocytes helpers of type1, which are responsible for the development of specific antitumoral immunity reactions.

Jerne N., Nordin A. (1963) Plaque formation in agar by single antibody production cells.

Science, V. 140, P. 405.

Kitamura K. (1980) A foot pad weight assay to evaluate delayed type hypersensitivity in the mice. J.Immunol. Meth., V.39, P. 277-283.

### Example 7

Influence of thymus gland basic glycoprotein on survival of mice with thymoma EL 4.

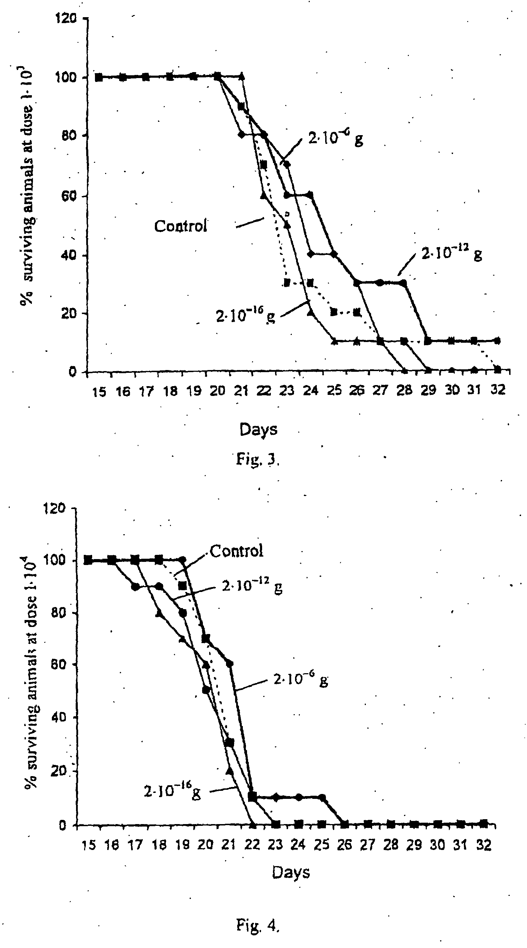

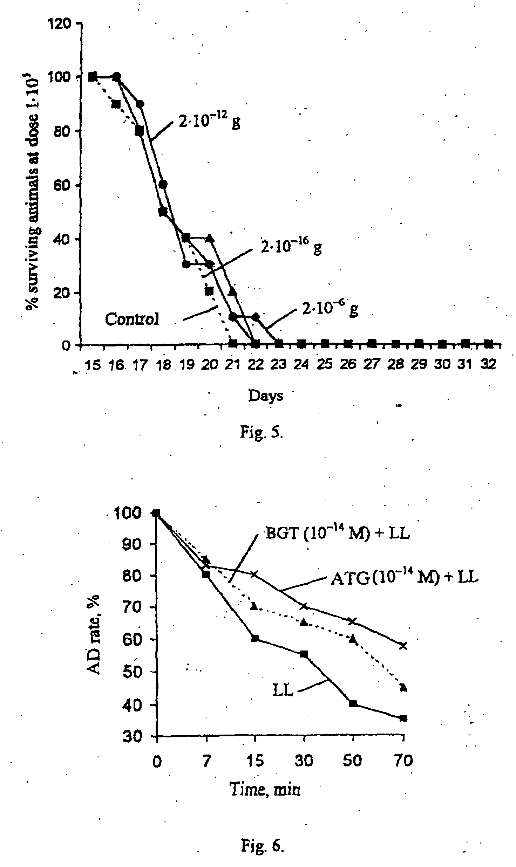

BTG has been studied to establish if it has an activity stimulating antitumoral immunity. With this purpose, mice of C57BL/6 $K^b I^b D^b$ received one injection of thymoma EL 4 cells, occurring from mice of the same line, in doses $1 \times 10^3$, $1 \times 10^4$ and $1 \times 10^5$. The specimen was introduced daily within one week in doses $2 \times 10^{-6}$, $2 \times 10^{-12}$ and $2 \times 10^{-16}$ g for one animal, since the day of immunization (nine test groups and 3 control groups, each of 5-7 animals). Effect of the specimen was evaluated according to life expectancy of the recipients of various tumor cells doses, compared to the appropriate groups of the control recipients. Results are shown on Figures 3-5. Figure 3 shows protective effect of the specimen in dose of $2 \times 10^{-12}$ g for one animal (thick line) in comparison with the Control (dotted line). In this Test group 1 of 10 recipients remained alive, whereas in the Control all the animals died as a result of tumor grows in ascitic form. Figure 4 shows low protective effect of the specimen in dose of $2 \times 10^{-6}$ g for one animal (thick line) in comparison with the Control (dotted line). When tumor cells dose was $1 \times 10^5$, it appeared impossible to see the effect of the specimen (fig. 5). The submitted data show that BTG in dose of $2 \times 10^{-12}$ g for one mouse, leads to statistically reliable augmentation of life expectancy in test animals - group, received minimum dose of tumor cells ($1 \times 10^3$).

In spite of the fact that biological effect, displayed by BTG is insignificant, the following should be taken into account: time of thymoma cells duplication exceeds time of Ò-lymphocytes with antitumoral effector activity duplication in several times. Besides, complete rejection of inserted tumor cells is observed, as a rule, only when the inserted tumor is modified with genetic structure for cytokine production, which stimulate cellular immunity, or under combined therapy with cytostatics.

Sumimoto H., Tani K., Nakazaki Y., Tanabe T., Hibino H., Wu M.S., Izawa K., Hamada H., Asano S. (1998) Superiority of interleukin-12-transduced murine lung cancer cell to GM-CSF or B7-1 (CD 80) transfectants for therapeutic antitumoral immunity in syngeneic immunocompetent mice. Cancer Gene Ther V. 5, N 1, P.29-37.

Ehrke M.J., Verstovsek S., Pocchiari S.K., Krawczyk C. M., Ujhazy P., Zaleskis G., Maccubbin D.L., Meer J. M., Mihich E. (1998) Thymic anti-tumor effectors in mice cured of lymphoma by cyclophosphamide plus TNF-alpha therapy: phenotypic and functional characterization up to 20 months after initial tumor inoculation. Int. J. Cancer, V. 76, N. 4, P 579-586.

Example 8.

Influence of thymus gland acidic glycoprotein on delayed-type hypersensitivity reaction

Acidic thymus glycoprotein (ATG) was administered to four groups of 5 mice with C57BL/6 spf status, with weight 20 g. Intraperitoneal introduction was made three times in doses of $2 \times 10^{-6}$, $2 \times 10^{-12}$, $2 \times 10^{-16}$ and $2 \times 10^{-18}$ g for one animal within three days, from the moment of intraperitoneal immunization of mice with sheep erythrocytes in dosage of $2 \times 10^8$ for one animal. In 5 days after immunization, $1 \times 10^8$ of sheep erythrocytes were administered to the recipients in 50 µl subcutaneously in back leg's foot. And in one day more, the intensity of edema was defined in comparison with counter-lateral extremity by Kitamura method, and number of spleen antibody-forming cells was defined by Jeme method. The results are shown in table 2.

It is well seen from the table, that the specimen doesn't exert a great influence on antibody-forming cells formation in spleen. At the same time, the specimen introduction in dose of $2 \times 10^{-12}$ g for one animal results in highly reliable inhibition of delayed-type hypersensitivity reaction.

Thus, we show the ability of ATG specimen in dose of $2 \times 10^{-12}$ g for one animal to slow down delayed-type hypersensitivity reaction.

Table 2

| Influence of thymus gland acidic glycoprotein on antibody-forming and delayed-type hypersensitivity reaction in mice of C57BL/6 line | | |
| --- | --- | --- |
| Test groups | Delayed-type hypersensitivity reaction (%) | Antibody-forming cells to spleen |
| The Control | $25{,}2 \pm 1{,}61$ | $5900 \pm 1500$ |
| ATG, $2 \times 10^{-6}$g | $25{,}22 \pm 2{,}5$ | $4850 \pm 1200$ |
| ATG, $2 \times 10^{-12}$g | $17{.}64 \pm 1{,}52^*$ | $4130 \pm 1400$ |
| ATG, $2 \times 10^{-16}$g | $22{,}5 \pm 1{,}44$ | $5150 \pm 780$ |
| ATG, $2 \times 10^{-18}$g | $26{,}4 \pm 1{,}2$ | $5550 \pm 600$ |

The results represent arithmetic average ± average mistake.
\* - Reliable distinctions from the Control, Ð < 0.001

Example 9.

Influence of glycoproteins from mammalian thymus gland on excitement conduction under demyelinization of nerve fiber

As the object of research myelinic nerves of grass frog (Rana tamporaria) have been taken. To perform a focal demyelinization of nerves in vivo, lysolecithin (LL) was used, which was introduced into the sheath of myelinic ischiatic nerve of the frog at operation (intracavitary injection). It is known, that this action initiates nerve demyelinization, several stages of which correspond with autoimmune demyelinization. First changes of myelin condition are observed in a day after the operation, and in 6-12 days myelin completely "uncover" the internodal parts of nerve fiber. In 3-4 weeks after the operation new myelin formation is observed.

The study of influence of basic and acidic thymus glycoproteins on the processes of demyelinization and regeneration after demyelinization were carried out in two experiments: in vitro, on the isolated nerve, and also in vivo, in one week after the introduction of LL to the animals. LL and the studied thymus glycoprotein were administered to the animals simultaneously. When conducted in vivo experiments, the following tests were made:

1. Influence of LL and thymus glycoprotein on the electrophysiological parameters of a nerve was evaluated.
2. The effect of combined action of LL and thymus glycoprotein was determined.

The control animals were not exposed to LL and thymus glycoprotein influence, but were placed in the same conditions during all time of the experiment. During in vitro experiment, standard method of extracellular registration of membrane potential and action potential (AD) was used. It helped to determine:

1. Change dynamics of AD amplitude, threshold, AD rate of conduction, and maximal frequency of the isolated nerve rhythmic answer under LL action, thymus glycoproteins action and their combined action.
2. Change dynamics of AD amplitude, threshold, AD rate of conduction, and maximal frequency of the isolated nerve rhythmic answer in one week after combined introduction of LL and thymus glycoproteins to the animal.

Glycoproteins from thymus gland were studied in concentrations $10^{-14}$ mol/l, $10^{-18}$ mol/l and $10^{-24}$ mol/l.

Isolated nerves were previously placed for at least 30 minutes in physiological solution of the following composition (mM): NaCl - 111.2; ÊÑI -1.88; $CaCl_2$ -1.08; pH - 7.2; 18-20°C. To prepare physiological solution and solutions of glycoproteins, prepared on physiological solution of the specified composition, redistillate was used.

To evaluate the changes of membrane-bound calcium level, in this research we used a method of fluorescent spectroscopy with application of localized in plasma membrane $Ca^{2+}$-binding probe - chlortetracycline, which is capable to form a complex with calcium ion. The experiments on nerves were carried out in a specially designed chamber, which allows recording simultaneously the level of nerve fluorescence and AD amplitude. Luminescence was recorded from the same nerve part or isolated fiber during all experiment. Nerve fluorescence was recorded with the help of Lumam 1-3 (LOMO) luminescent microscope. Fluorescence stimulation of chlortetracycline was caused by halogen lamp KGM 9õ870 and combination of filters PS-1-6 and SZS 21-2.

Registration was carried out with the help of a photometric nozzle and interferential light filters with wavelength of maximal light transmission at 490 and 550 nm.

Diameter of nerve part photometry was 50 microns, at an objective õ10.

Under LL action and nerve demyelinization, significant changes in some electrophysiological parameters of rhythmic neurility (RN) occur. During present research we found out, that in vivo basic and acidic thymus glycoproteins in studied dose compensated changes of RN parameters in nerve demyelinization (Tables. 3, 4 and 5).

It is necessary to note, that injection of glycoprotein solutions alone did not result in reliable changes in myelinic neurility.

Table 3

| The study of lysolecithin and thymus glycoproteins combined influence on RN of demyelinizated nerve (concentration of glycoproteins was $10^{-14}$ Ì) | | |
|---|---|---|
| In vivo | Time of AD conduction (% to the Control) | AD amplitude (% to the Control, 100 Hz) |
| LL | 160 | 30 |
| Acidic glycoprotein from thymus gland | 130 | 66 |
| Basic glycoprotein from thymus gland | 140 | 40 |

In separate experiment the restoration of neurility in the whole animal after injection of lower concentration of thymus glycoproteins ($10^{-18}$ Ì and $10^{-24}$ Ì) was investigated. The results of this research are shown in tables 4 and 5.

Table 4

| The study of lysolecithin and thymus glycoproteins combined influence on RN of demyelinizated nerve (concentration of glycoproteins was $10^{-18}$ Ì) | | |
| --- | --- | --- |
| In vivo | Time of AD conduction (% to the Control) | AD amplitude (% to the Control, 50 Hz) |
| LL | 160 | 30 |
| Acidic glycoprotein from thymus gland | 150 | 38 |
| Basic glycoprotein from thymus gland | 155 | 35 |

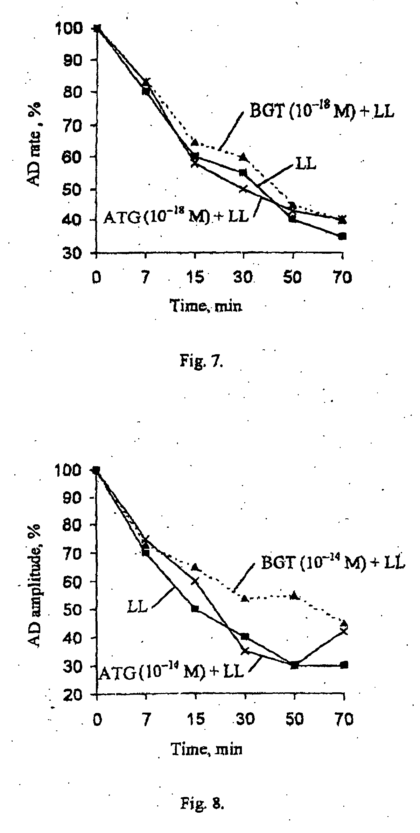

It is well known, that the destruction of myelin results in rate reduction of AD extension. During this research, we found out that under LL action, rate of AD conduction by isolated nerves was reduced, and under combined action of LL and studied thymus glycoproteins the character of these changes was decreased (Fig. 6, 7). Under maximal dilution of thymus glycoprotein solutions (to $10^{-24}$ Ì ), just tendency to restoration in the first 7-15 mines of LL and glycoprotein solution incubation was observed (but there were no reliable differences). Thus, long incubation of nerves in solutions of thymus glycoproteins and LL, results in statistically reliable restoration of AD conduction rate only in case of $10^{-14}$ mol/liter concentration.

Table 5

| The study of lysolecithin and thymus glycoproteins combined influence on RN of demyelinizated nerve (concentration of glycoproteins was $10^{-24}$ Ì) | | |
| --- | --- | --- |
| In vivo | Time of AD conduction (% to the Control) | AD amplitude (% to the Control, 50 Hz) |
| LL | 160 | 30 |
| Acidic glycoprotein from thymus gland | 155 | 35 |
| Basic glycoprotein from thymus gland | 158 | 32 |

In the following series of experiments changes of AD amplitude under LL and thymus glycoproteins action were investigated (fig. 8). It was determined, that during the time of the experiment (LL action), significant changes of this RN parameter were observed (at frequency of 100 Hz). Under combined action of LL and thymus glycoproteins ($10^{-14}$ Ì) the revealed changes of AD amplitude are restored.

In the following series of experiments redistribution intercellular calcium was studied, recording fluorescence of a probe - chlortetracycline, which allowed revealing the localization and changes of membrane-bound calcium level. Fluorescence of nerves was investigated, as well as nerve fibers incubated in LL solution, and fibers under combined action of LL and thymus glycoproteins.

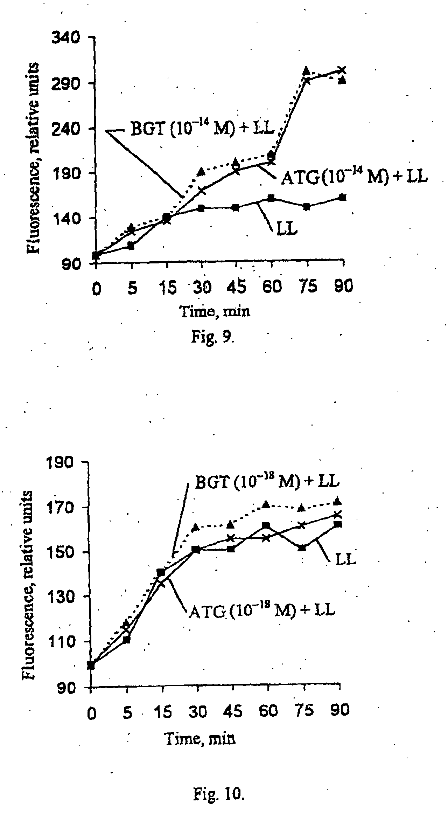

It was earlier shown, that when a nerve was incubated with chlortetracycline, maximal value of fluorescence - the parameter proportional to membrane-bound $Ca^{2+}$ level - was recorded from membrane nerve structures (plasma membranes of an axon and Schwann cell, and also myelin). Under LL action, membrane-bound calcium levels increases, but under combined action of LL and thymus glycoproteins value of binding increases much more (fig. 9-11). As it follows from the results obtained, maximal value of calcium binding is significantly changed under LL and thymus glycoproteins action in concentration $10^{-14}$ M. As far as concentration of thymus glycoproteins is decreased to $10^{-18}$ Ì , maximal calcium binding by membrane nerve structures is reduced, but it exceeds the Control (action of LL). The decrease of thymus glycoprotein concentration to $10^{-24}$ M during 90 minutes resulted to small changes of bound calcium level. The obtained data show the ability of studied glycoproteins to change a level of membrane-bound calcium in investigated "imaginary" solutions.

The research has revealed changes of several parameters, describing RN, in case of nerve demyelinization, and also the presence of characteristic RN reorganizations under thymus glycoprotein's action both in vivo, and in vitro.

Kols O.P., Maksimov G.V., Radenovich Ch.N. Biophysics of rhythmic neurility, Moscow, Moscow State University, 206,1993

Maksimov G.V., Orlov S.N. Transport of calcium ions while nerve fiber functioning: mechanisms and regulation. Moscow, Moscow State University, 88, 1994

Waxman S.G., Kocsis J.D., Stys P.K. The axon. Structure, function and pathophysiology. Oxford Univ. Press., NY-Oxford, 325, 1995

Example 10

Influence of glycoprotein from mammalian blood serum (đI in đÍ interval 4.65-5.1) on excitation conduction in demyelinizated nerve fiber.

The experiment was conducted under the technique described in Example 9. Under LL action and nerve demyelinization, significant changes in some electrophysiological parameters of rhythmic neurility (RN) occur. During present research we found out, that in vivo serum glycoprotein (SG) in dose of $10^{-14}$ mol/liter compensated changes of RN parameters in nerve demyelinization (Table 6). It is necessary to note, that injection of glycoprotein solutions alone did not result in reliable changes in myelinic neurility.

Table 6

| The study of lysolecithin and serum glycoprotein combined influence on RN of demyelinized nerve in vivo | | |
|---|---|---|
| In vivo | Time of AD conduction (% to the Control) | AD amplitude (% to the Control) |
| LL (at 50 and 100 Hz) | 160 | 30 |
| Serum glycoprotein in dose of $10^{-14}$ mol/liter (at 100 Hz) | 80 | 55 |
| Serum glycoprotein in dose of $10^{-18}$ mol/liter (at 50 Hz) | 145 | 45 |
| Serum glycoprotein in dose of $10^{-24}$ mol/liter (at 50 Hz) | 145 | 32 |

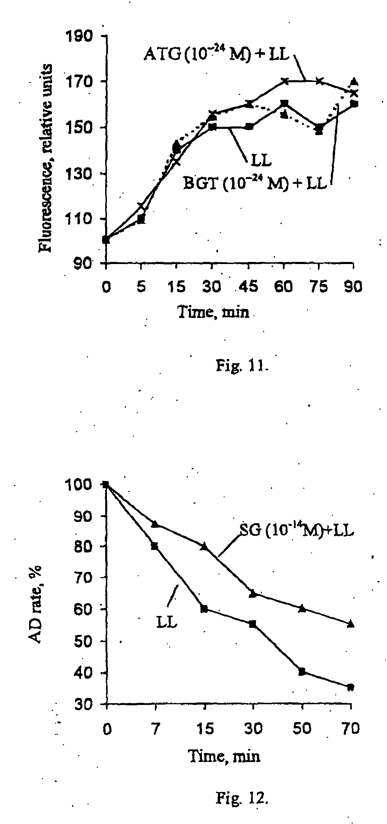

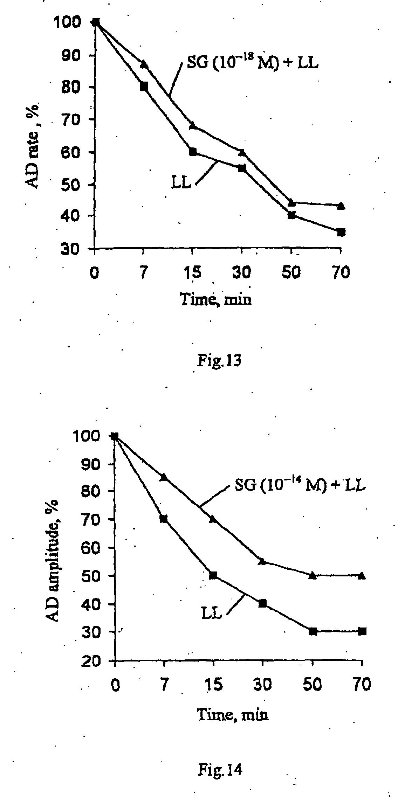

It is determined, that under combined LL and SG influence on a nerve, the character of changes, caused by introduction of LL, decreases (fig. 12, 13). When the SG solution was diluted to concentration of $10^{-18}$ mol/liter, biological effect was insignificant, but the data had reliable differences in comparison to the Control. Thus, long incubation of nerves in solutions of SG and LL, results in statistically reliable restoration of AD conduction rate in concentrations of $10^{-14}$ mol/liter and $10^{-18}$ mol/liter.

In the following series of experiments changes of AD amplitude under LL and SG actions were investigated (fig. 14). It was determined, that during the time of the experiment (LL action), significant changes of this RN parameter were observed (at frequency of 100 Hz). Under combined action of LL and SG ($10^{-14}$ Ì ) the revealed changes of AD amplitude are restored.

In the following series of experiments redistribution intercellular calcium was studied, recording fluorescence of a probe - chlortetracycline, which allowed revealing the localization and changes of membrane-bound calcium level. Fluorescence of nerves was investigated, as well as nerve fibers incubated in LL solution, and fibers under combined action of LL and SG.

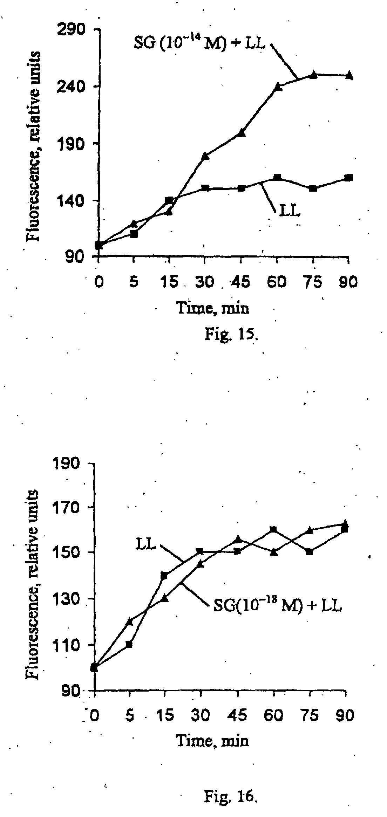

Under LL action, membrane-bound calcium levels increases, but under combined action of LL and SG value of binding increases much more (fig. 15-17). As it follows from the results obtained, maximal value of calcium binding is significantly changed under LL and SG action in concentration $10^{-14}$ M. As far as concentration of SG is decreased to $10^{-24}$ Ì , maximal calcium binding by membrane nerve structures is reduced. The obtained data show the ability of SG to change a level of membrane-bound calcium in investigated "imaginary" solutions.

The research has revealed changes of several parameters, describing RN, in case of nerve demyelinization, and also the presence of characteristic RN reorganizations under serum glycoprotein's action both in vivo, and in vitro.

Example 11

Influence of basic glycoprotein from bull retina on excitation conduction in demyelinizated nerve fiber.

The experiment was conducted under the technique described in Example 9.

Under LL action and nerve demyelinization, significant changes in some electrophysiological parameters of rhythmic neurility (RN) occur. During present research we found out, that in vivo basic glycoprotein from bull retina (BRG) in doses of $10^{-14}$ mol/liter and $10^{-18}$ mol/liter compensated changes of RN parameters in nerve demyelinization (Table 7). It is necessary to note, that injection of glycoprotein solutions alone did not result in reliable changes in myelinic neurility.

Table 7

| The study of lysolecithin and basic glycoprotein from bull retina combined influence on RN of demyelinizated nerve in vivo | | |
|---|---|---|
| In vivo | Time of AD conduction (% to the Control) | AD amplitude (% to the Control) |
| LL (at 50 and 100 Hz) | 160 | 30 |
| BRG in dose of $10^{-14}$ mol/liter (at 100 Hz) | 145 | 45 |
| BRG in dose of $10^{-18}$ mol/liter (at 50 Hz) | 148 | 35 |
| BRG in dose of $10^{-24}$ mol/liter (at 50 Hz) | 156 | 30 |

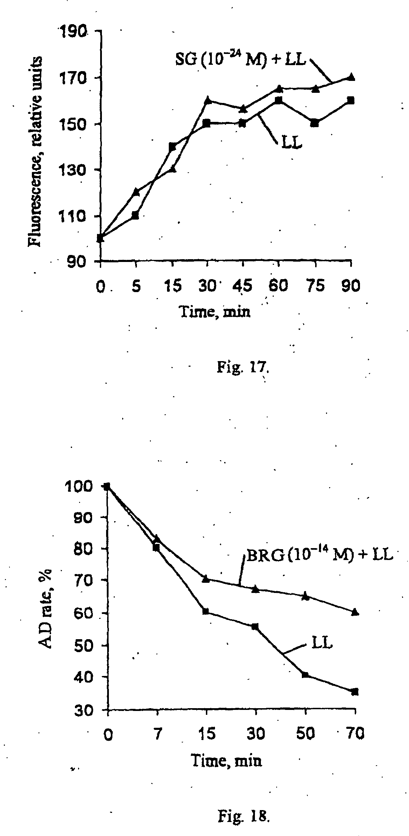

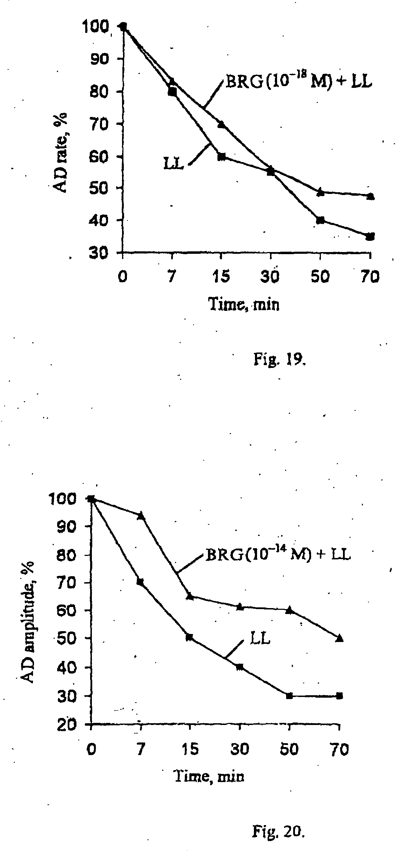

It is determined, that under combined LL and BRG influence on a nerve, the character of changes, caused by introduction of LL, decreases (fig. 18, 19). When the BRG solution was diluted to concentration of $10^{-18}$ mol/liter, value of biological effect had reliable differences in comparison to the Control. Thus, long incubation of nerves in solutions of BRG and LL, results in statistically reliable restoration of AD conduction rate in concentrations of $10^{-14}$ mol/liter and $10^{-18}$ mol/liter.

In the following series of experiments changes of AD amplitude under LL and BRG actions were investigated (fig. 20). It was determined, that during the time of the experiment (LL action), significant changes of this RN parameter were observed (at frequency of 100 Hz). Under combined action of LL and BRG ($10^{-14}$ Ì ) the revealed changes of AD amplitude are restored.

In the following series of experiments redistribution intercellular calcium was studied, recording fluorescence of a probe - chlortetracycline, which allowed revealing the localization and changes of membrane-bound calcium level. Fluorescence of nerves was investigated, as well as nerve fibers incubated in LL solution, and fibers under combined action of LL and BRG.

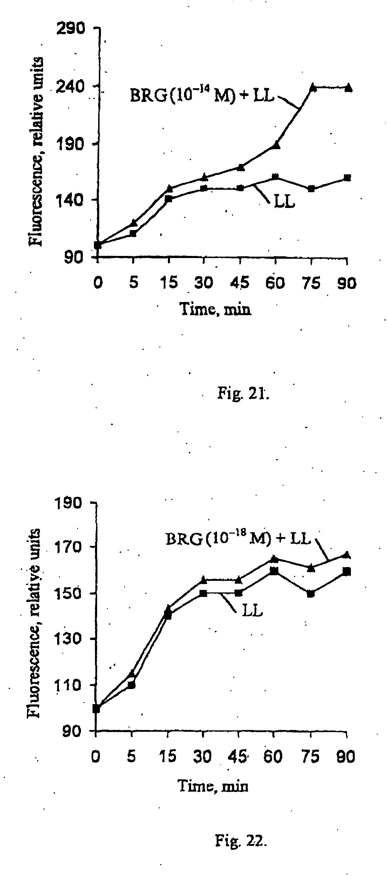

Under LL action, membrane-bound calcium levels increases, but under combined action of LL and BRG value of binding increases much more (fig. 21-23). As it follows from the results obtained, maximal value of calcium binding is significantly changed under LL and BRG action in concentration $10^{-14}$ M. As far as concentration of BRG is decreased to $10^{-24}$ Ì , maximal calcium binding by membrane nerve structures is reduced. The obtained data show the ability of BRG to change a level of membrane-bound calcium in investigated "imaginary" solutions.

The research has revealed changes of several parameters, describing RN, in case of nerve demyelinization, and also the presence of characteristic RN reorganizations under basic glycoprotein from bull retina's action both in vivo, and in vitro.

Example 12

Influence of basic glycoprotein from bull's eye pigmented epithelium on excitation conduction in demyelinizated nerve fiber.

The experiment was conducted under the technique described in Example 9.

Under LL action and nerve demyelinization, significant changes in some electrophysiological parameters of rhythmic neurility (RN) occur. During present research we found out, that in vivo basic glycoprotein from bull's eye pigmented epithelium (BPEG) in dose of $10^{-14}$ mol/liter compensated changes of RN parameters in nerve demyelinization (Table 8). It is necessary to note, that injection of glycoprotein solutions alone did not result in reliable changes in myelinic neurility.

Table 8

| The study of lysolecithin and basic glycoprotein from bull's eye pigmented epithelium combined influence on RN of demyelinizated nerve in vivo | | |
| --- | --- | --- |
| In vivo | Time of AD conduction (% to the Control) | AD amplitude (% to the Control) |
| LL (at 50 and 100 Hz) | 160 | 30 |
| BPEG in dose of $10^{-14}$ mol/liter (at 100 Hz) | 145 | 40 |
| BPEG in dose of $10^{-18}$ mol/liter (at 50 Hz) | 155 | 38 |
| BPEG in dose of $10^{-24}$ mol/liter (at 50 Hz) | 160 | 30 |

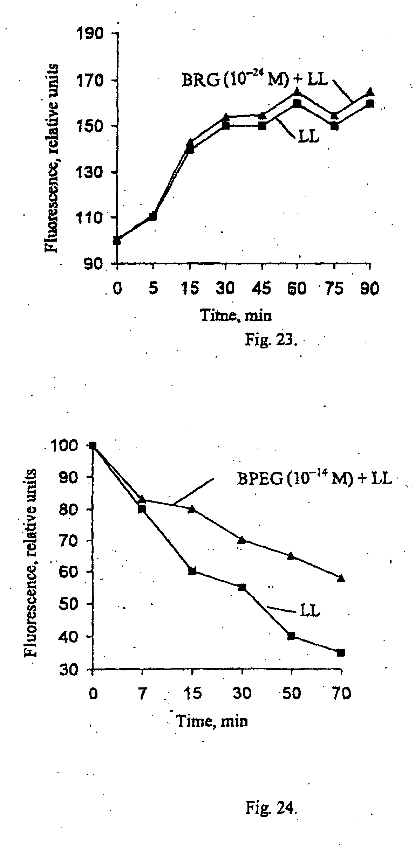

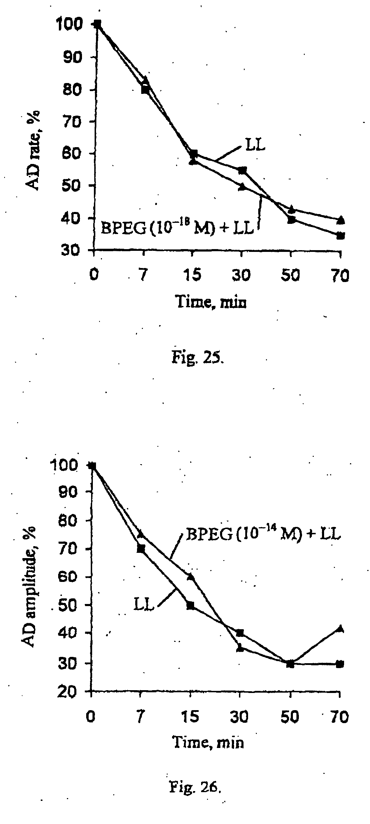

It is determined, that under combined LL and BPEG influence on a nerve, the character of changes, caused by introduction of LL, decreases (fig. 24, 25). Long incubation of nerves in solutions of BPEG and LL, results in statistically reliable restoration of AD conduction rate in concentration of $10^{-14}$ mol/liter.

In the following series of experiments changes of AD amplitude under LL and BPEG actions were investigated (fig. 26). It was determined, that during the time of the experiment (LL action), significant changes of this RN parameter were observed (at frequency of 100 Hz). Under combined action of LL and BPEG ($10^{-14}$ Ì ) the revealed changes of AD amplitude are restored.

In the following series of experiments redistribution intercellular calcium was studied, recording fluorescence of a probe - chlortetracycline, which allowed revealing the localization and changes of membrane-bound calcium level.

Fluorescence of nerves was investigated, as well as nerve fibers incubated in LL solution, and fibers under combined action of LL and BPEG.

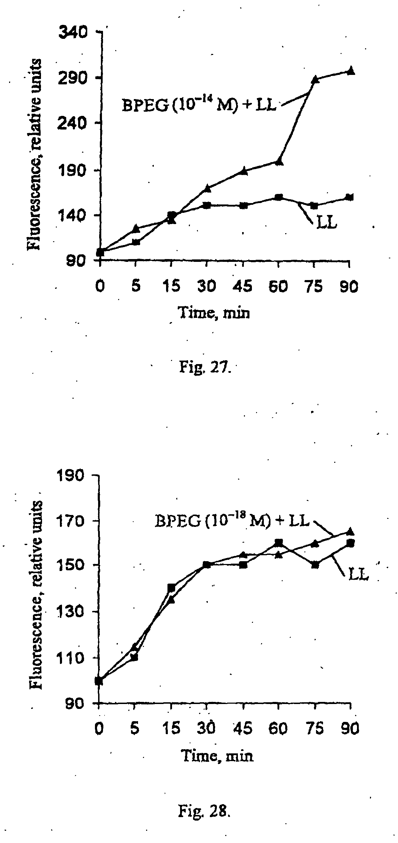

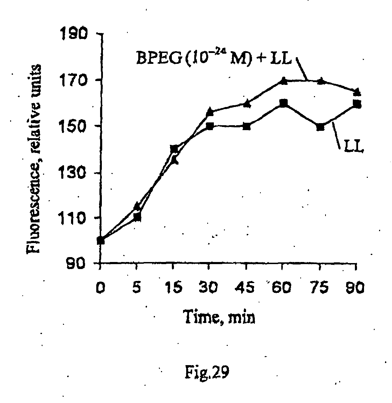

Under LL action, membrane-bound calcium levels increases, but under combined action of LL and BPEG value of binding increases much more (fig. 27-29). As it follows from the results obtained, maximal value of calcium binding is significantly changed under LL and BPEG action in concentration $10^{-14}$ M. As far as concentration of BPEG is decreased to $10^{-18}$ and $10^{-24}$ Ì , maximal calcium binding by membrane nerve structures is reduced. The obtained data show the ability of BPEG to change a level of membrane-bound calcium in "imaginary" ($10^{-14}$ mol/liter) solutions.

The research has revealed changes of several parameters, describing RN, in case of nerve demyelinization, and also the presence of characteristic RN reorganizations under basic glycoprotein from bull's eye pigmented epithelium action both in vivo, and in vitro.

Example 13

Influence of acidic liver glycoprotein on plasma membrane permeability of hepatocytes and on protein synthesis intensity in vitro

The experiment was carried out on multiple organ liver culture taken from rats of Wistar line, males with weight of 180 g. 5 experiments were conducted. 25-30 pieces of liver were used in each experiment. All explantats studied in one experiment were received from liver of one animal. Explantats were cultivated on liquid nutrient medium and air interface, using millipore filters (Synpor, with 0.6 mm in diameter). Cultivation was carried out for 14-16 hours at 37°C. After cultivation, a group of explantats was transferred for 10 minutes to culture medium, containing labeled $^3$H-leucine in dose of 25 µCu. Intensity of protein synthesis was evaluated on leucine inclusion in proteins referred to the labeled leucine pool in the same sample.

Permeability of cells for labeled precursor was determined by the pool of free labeled amino acid. Influence of acidic liver glycoprotein (ALG) was defined for its solution in concentration of $10^{-14}$ mol/liter. Solution of ALG was added to in culture medium in test vials 2 hours prior to incubation with labeled leucine, and then for 10 minutes it was transferred to the medium containing ALG and labeled amino acid in the specified concentrations.

It was found out that intensity of protein synthesis under ALG influence was considerably reduced in comparison with the control, approximately twice less (in the Control, ratio of the labeled leucine inclusion to the pool of this precursor made, for example, $0,42\pm0,09$, and in the Test - $0,21\pm0,04$).

Permeability of hepatocyte membrane was increased considerably at presence of ALG (in the Control, for example, the pool of free amino acids made 1120±50 imp. per minute, and in test vials correspondingly 2180±30).

Morphology of explantats from the test organ culture was not changed in comparison with the control.

The results of the conducted research show, that ALG influences the intensity of protein synthesis in hepatocytes and permeability of their plasma membranes in ultra low dose under condition of retention of liver tissue structure.

Example 14

Influence of glycoprotein from mammalian blood serum (đI in đÍ interval 4.65-5.1) on plasma membrane permeability of hepatocytes and on protein synthesis intensity in vitro

The experiment was carried out on multiple organ liver culture taken from rats of Wistar line, males with weight of 180 g. 5 experiments were conducted. 25-30 pieces of liver were used in each experiment. All explantats studied in one experiment were received from liver of one animal. Explantats were cultivated on liquid nutrient medium and air interface, using millipore filters (Synpor, with 0.6 mm in diameter). Cultivation was carried out for 14-16 hours at 37°Ñ. After cultivation, a group of explantats was transferred for 10 minutes to culture medium, containing labeled $^3$H-leucine in dose of 25 μCu. Intensity of protein synthesis was evaluated on leucine inclusion in proteins referred to the labeled leucine pool in the same sample. Permeability of cells for labeled precursor was determined by the pool of free-labeled amino acid. Influence of serum glycoprotein (SG) was defined for its solution in concentration of $10^{-14}$ mol/liter. Solution of SG was added to in culture medium in test vials 2 hours prior to incubation with labeled leucine, and then for 10 minutes it was transferred to the medium containing SG and labeled amino acid in the specified concentrations.

It was found out that intensity of protein synthesis under SG influence was considerably reduced in comparison with the control, approximately twice less (in the Control, ratio of the labeled leucine inclusion to the pool of this precursor made, for example, 0,42±0,09, and in the Test - 0,17±0,05). Permeability of hepatocyte membrane was increased considerably at presence of SG (in the Control, for example, the pool of free amino acids made 1120±50 imp. per minute, and in test vials correspondingly 2980±30).

Morphology of explantats from the test organ culture was not changed in comparison with the control.

The results of the conducted research show, that SG influences the intensity of protein synthesis in hepatocytes and permeability of their plasma membranes in ultra low dose under condition of retention of liver tissue structure.

The following examples (15-17) describe pharmaceutical composition based on glycoprotein under this [given] invention.

Example 15

Composition based on glycoprotein from mammalian blood serum (đI in đÍ interval 4.65-5.1) in ultra low doses, having pharmacological action.

| Composition: | |
|---|---|
| Serum glycoprotein | $1 \times 10^{-10}$ g |
| Sodium chloride | 8.8 g |
| Calcium chloride | 0.001 g |
| Water | up to 1 liter |

is a bioregulator of reparative processes in epithelial and connecting tissues.

When used as eye drops, this specified composition assists in cornea healing after a mechanical trauma or combustion. It causes formation of mild scar, limiting at the same time the excessive growth of scar tissue. It is especially effective at keratoplasty, treatment of keratites and some conjunctivitis. Application of this composition to treatment of cornea penetrating wounds, results in the decrease of inflammatory reaction terms, fast liquidation of wound edges diastasis, acceleration of damage surface epithelization, earlier regeneration of the front chamber, reduction of complication frequency (effusion of fibrine and hypopyon), acceleration of reparative regeneration and reconstruction of new scar tissue, which leads to formation of more compact and structured scar with prevalence of proliferative component without active vascularization of cornea. On cellular level is seen the following: the acceleration of migration of epithelium, macrophages, keratoblasts, the decrease of leukocytic infiltration, early formation of continuous layer of endothelia without sings of desquamation in a wound canal, that finally results in fast filling of wound canal with epithelial-fibrinous component, earlier and exact closure of wound edges, active resorption of fibrin and its replacement by keratoblastic proliferate, which is synthesizing new collagen fiber. When this composition is used, the following happens: more compact arrangement of collagen plates and prevalence of fibrous component of proliferate above cellular, which determines quality of scar tissue. The formation of more gentle, compact and nonvascular scars results in smaller changes of corneal transparence and refraction. In case of eye burn therapeutical effect of the specified composition develops to the 14th day and consists of the following: significant proliferation of fibroblastic elements, infiltrating injured comeal tissue, and vector orientation of proliferating cells causes plate structure of cornea imitating the initial morphological tissue structure.

When used as injection form, this specified composition stimulates bone tissue regeneration in fractures of extremities, including cervical hip fracture. It is effective for treatment of serious articular pathologies, associated with structural and functional abnormalities of cartilage tissue, for treatment of mechanical damages of articular knee cartilage, for treatment of arthroses and synovitis. The introduction of the composition inhibits degeneration process of damaged cartilage tissue. When used for treatment of damaged articular cartilage, this composition provides fast accumulation of young cartilaginous cells and their differentiation, resulting in faster, than in the control, formation of substituting cartilage tissue and reconstruction of even articular surface in damage area, which, in its turn, plays the basic role in restoration of articular mobility. Young cartilaginous cells fill the area of damage, and cartilage tissue regenerates in definitive way, because its differentiation on layers like the initial hyaline cartilage takes place. When intra-articular introduction of the composition is carried out, the decrease of pain symptoms is observed after the 2-3 injection of a drug. If the application efficiency of the specified composition is compared to the most known chondroprotectors, for example "Zeel", in case of treatment for traumatic damages of articular knee cartilage in the sportsmen, it is possible to note, that pain symptoms and synovitis was stopped on the 7-10 day in the average in case of application of the given composition, and on the 14-17 day in case of "Zeel" application.

Hence, the restoration of sports results to a former level occurred 2-2.5 times faster, than in case of "Zeel" application.

Various compositions containing, as an active component, serum glycoprotein in concentration of $1 \times 10^{-10}$ g of glycoprotein/liter (or kg) of composition in various medicinal forms (gels, ointments, suppositories, solution) have wound healing activity and are effective for skin injures treatment, including the treatment of a burn disease, decubituses and their prevention. They stimulate skin reparative processes after radiation injury, occurring in the oncology patients after radiotherapy. These compositions are effective in gastroenterology (ulcer, gastritis, gastroduodenitis), in proctology (rectal diseases), in gynecology (cervical erosion), in cardiology (rehabilitation period after myocardial infarction).

Example 16

Composition based on acidic liver glycoprotein from cattle retina in ultra low doses, having pharmacological action.

| Composition: | |
| --- | --- |
| Acidic glycoprotein from retina | $1 \times 10^{-10}$ g |
| Sodium chloride | 8.8 g |
| Calcium chloride | 0.001 g |
| Water | up to 1liter |

is a bioregulator of reparative processes, assisting in restoration of broken retina function, prevents retina exfoliation at surgical interventions.

This specified composition stimulates the functioning of the basic ferment systems in retina responsible for vision realization, inhibits peroxide oxidation of lipids in cell membranes of retina. The composition is an effective bioregulator, which is responsible for positional cell disposition in histological structure of retina and cell division. It assists in restoration of spatially - function organization of retina tissue after injury or pathological process development. This given composition has therapeutical effect in myopia disease, retina degeneration of various etiologies, and condition after eye penetrating wounds.

Example 17

Composition based on acidic glycoprotein from cattle eye pigmented epithelium in ultra low doses, having pharmacological action.

| Composition: | |
|---|---|
| Acidic glycoprotein from eye pigmented epithelium | $1 \times 10^{-10}$ g |
| Sodium chloride | 8.8 g |
| Calcium chloride | 0.001 g |
| Water | up to 1 liter |

is a bioregulator, assisting in restoration of eye pigmented epithelium broken function.

Nowadays, there are no pharmacological agents, application of which would cause inhibition of pathological process on initial stages of development of various etiology retinites and maculopathy. The specified composition is effective for treatment of maculopathy and retinites of various etiologies.

**Claims**

1. Glycoproteins, extracted with the help of isoelectric focusing from intercellular space of tissues taken from different organs, blood serum and bile of human beings and animals, that are soluble in saturated (100%) solution of ammonium sulphate, having apparent molecular weight of 10-45 kDa and having biological activity in ultra low doses from $10^{-12}$ to $10^{-29}$ mol/liter and lower.

2. Pharmaceutical composition, including glycoprotein of claim 1 in effective amount and pharmaceutically acceptable carrier.

3. Use of glycoprotein of claim 1 as a medicinal agent.

−lgC [mol/l] glycoprotein SG

Fig. 1.

Fig. 2.

Fig. 3.

Fig. 4.

Fig. 5.

Fig. 6.

Fig. 7.

Fig. 8.

Fig. 9.

Fig. 10.

Fig. 11.

Fig. 12.

Fig.13

Fig.14

Fig. 15.

Fig. 16.

Fig. 17.

Fig. 18.

Fig. 19.

Fig. 20.

Fig. 21.

Fig. 22.

Fig. 23.

Fig. 24.

Fig. 25.

Fig. 26.

Fig. 27.

Fig. 28.

Fig.29

**INTERNATIONAL SEARCH REPORT**

| International application No. |
| --- |
| PCT/RU 00/00295 |

| A. CLASSIFICATION OF SUBJECT MATTER |
| --- |
| **IPC 7 :**  A61K 38/17, 35/12, 35/14, 35/407 |
| According to International Patent Classification (IPC) or to both national classification and IPC |

| B. FIELDS SEARCHED |
| --- |
| Minimum documentation searched (classification system followed by classification symbols) |
| **IPC 7 :**  A61K 35/12, 35/14, 35/407, 38/17 |
| Documentation searched other than minimum documentation to the extent that such documents are included in the fields searched |
| Electronic data base consulted during the international search (name of data base and, where practicable, search terms used) |

| C. DOCUMENTS CONSIDERED TO BE RELEVANT | | |
| --- | --- | --- |
| Category* | Citation of document, with indication, where appropriate, of the relevant passages | Relevant to claim No. |
| Y | RU 2136695 C1 (ZAOPP "ENDO-FARM-A") **9 September 1999 (09.09.99)** <br> the claims, the description, page 2, examples 1,9 | 1-3 |
| Y | US 4559230 A ( INSTITUT PASTEUR ) **17 December 1985 (17.12.85)** <br> the claims, the example | 1-3 |
| A | US 4169139 A ( ARTHUR KARLER ) **25 September 1979 (25.09.79)** | 1-3 |
| A | EP 0228357 A2 ( WASHIGTON UNIVERSITY ) **8 July 1987 (08.07.87)** | 1-3 |
| A | GB 2097647 A ( CORNELL RESEARCH FOUNDATION INC.) <br> **10 November 1982 (10.11.82)** | 1-3 |
| A | RU 2078345 C1 (ANIKEEVA SVETLANA PETROVNA) <br> **27 April 1997 (27.04.97)** | 1 |

| ☐ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **2 April 2001 (02.04.01)** | **5 April 2001 (05.04.01)** |
| Name and mailing address of the ISA/ <br><br> **RU** <br> Facsimile No. | Authorized officer <br><br><br> Telephone No. |

Form PCT/ISA/210 (second sheet) (July 1992)